# EUROPEAN PATENT SPECIFICATION

(11) **EP 2 335 708 B1**
(45) Date of publication and mention of the grant of the patent: **09.10.2013**
(21) Application number: 10192809.1
(22) Date of filing: 26.11.2010
(51) Int. Cl.: A61K 31/726, A61K 31/727, A61P 7/06

(54) **Sulphated glycosaminoglycans, including heparin and derivatives thereof, for use in inhibiting the expression of hepcidin and for the therapeutic treatment of anaemia with high levels of hepcidin**
Sulfatierte Glycosaminoglykanen,einschliesslich Heparin und seine Derivate, zur Inhibition der Expression von Hepcidin und zur Behandlung von Anämie mit erhöhtem Hepcidin-Spiegel
Glycosaminoglycannes sulfatés, comprenant de l'héparine ou des derivés de celle-ci, pour l'inhibition de l'expression de l'hepcidine et pour le traitement de l'anémie avec un taux de hepcidine élevé

(30) Priority: 01.12.2009 IT TO20090940
(43) Date of publication of application: 22.06.2011
(73) Proprietor: Universita' degli studi di Brescia, 25121 Brescia (IT)
(72) Inventor: Arosio, Paolo, I-25121, BRESCIA (IT); Poli, Maura, I-25121, BRESCIA (IT)
(74) Representative: Comoglio, Elena

(56) References cited:
- DATABASE WPI Week 199128 Thomson Scientific, London, GB; AN 1991-205653 XP002588481, & SU 1 563 697 A1 (OBSTETRICS GYNAECOL) 15 May 1990 (1990-05-15)
- YU LU ET AL: "Novel hollow microcapsules based on iron-heparin complex multilayers.", LANGMUIR : THE ACS JOURNAL OF SURFACES AND COLLOIDS 2 DEC 2008 LNKD- PUBMED:18855487, vol. 24, no. 23, 2 December 2008 (2008-12-02), pages 13723-13729, XP002588472, ISSN: 0743-7463
- ROTH K L ET AL: "Effect of intramuscular heparin on antibodies in idiopathic acquired hemolytic anemia", AMERICAN JOURNAL OF MEDICINE, EXCERPTA MEDICA, INC, UNITED STATES LNKD- DOI:10.1016/0002-9343(56)90264-9, vol. 20, no. 6, 1 June 1956 (1956-06-01), pages 968-970, XP026457216, ISSN: 0002-9343 [retrieved on 1956-06-01]
- BRUGNARA CARLO: "An immunoassay for human serum hepcidin at last: Ganz klar?", BLOOD 15 NOV 2008 LNKD- PUBMED:18988874, vol. 112, no. 10, 15 November 2008 (2008-11-15), pages 3922-3923, XP002628273, ISSN: 1528-0020

## Description

The present invention relates to a new therapeutic application of sulphated glycosaminoglycans.

Sulphated glycosaminoglycans (also known as glycosaminoglycan sulphides or sulphates), more in particular heparin and low molecular weight heparins, are high affinity binding molecules of numerous extracellular substances. It has been demonstrated that these molecules are potentially involved in biological processes such as angiogenesis, viral invasion, tumour growth and bone metabolism.

Heparin is a natural carbohydrate which belongs to the glycosaminoglycan family. It is composed of repeating units of disaccharides, hyduronic acid and N-glucosamine, which bind sulphate groups to nitrogen and oxygen atoms. Commercial heparin has an average molecular weight of 13,000 -15,000 Dalton, and low molecular weight heparins are obtained by heparin depolymerisation. Heparin derivatives are glycosaminoglycans of natural origin, such as heparan sulphates, or biotechnological origin having a different degree of sulphation and which may have reduced anticoagulant activity or no anticoagulant activity.

As is known, heparin and the derivatives thereof have been used for a long time as anticoagulant medicaments on account of their ability to bind to the anti-thrombin III, which in turn inactivates thrombin, factor X and other proteases involved in blood clotting.

The present inventors have now surprisingly found that molecules belonging to the class of sulphated glycosaminoglycans, such as heparin and heparan sulphates, show an approximately 100-folds inhibitory activity of the expression of hepcidin in hepatic cell lines at pharmacological concentrations.

Hepcidin is a small peptide rich in cysteine which plays a central role in regulating systemic iron homeostasis. Its expression is mainly hepatic, it is induced by iron overloads and by inflammatory states and is reduced by hypoxia and iron deficiency. Hepcidin induces degradation of its receptor, ferroportin, the sole cellular exporter of iron. Therefore, an increase in hepcidin results in the reduction of both iron uptake by duodenal enterocytes and hemoglobin iron recycling by the macrophages. The reduction of the expression of hepcidin is associated with iron overloads observed in hereditary haemochromatosis of various origin, while an excess of hepcidin is associated with refractory anaemia. Chronic disease anaemia (or inflammatory anaemia) is of particular importance given its diffusion and is characterised by an excess of hepcidin induced by the state of inflammation. It is also worth pointing out that, as of today, treatment of this disorder is based mainly on the use of erythropoietin and is very expensive and often not effective.

Recent studies have shown that hemojuvelin is the main regulator of the expression of hepcidin and that it acts as a co-receptor of bone morphogenic proteins (BMPs). Of these the protein BMP6 seems to be the physiological ligand of hemojuvelin and plays a central role in the regulation mechanism of hepcidin.
Furthermore, document "DATABASE WPI Week 199128" and article "An immunoassay for human serum hepcidin at last: Ganz klar?" (by BUNGARA CARLO) jointly disclose that iron deficiency anaemia is characterized by low levels of hepcidin.
Also, the scientific work "Novel hollow microcapsules based on iron-heparin complex multilayers" (YU LU ET AL.) describes heparin as an alternative polysaccharide for administering iron, so as to enhance and prolong its anti-coagulant activity.
At last, the article "Effect of intramuscular heparin on antibodies in idiopathic acquired hemolytic anemia" (ROTH K L ET AL) teaches that heparin has been used for the treatment of haemolytic anaemia, and describes the use of heparin in Idiopathic Acquired Haemolytic Anaemia which is based on a putative strong anticomplementary activity of heparin.

On the basis of this knowledge, it may be stated that the aforementioned sulphated glycosaminoglycans are involved in binding the molecules not implicated in clotting- such as presumably the BMPs, their receptors, co-receptors or inhibitors- which play a role in regulating the expression of hepcidin and are therefore useful in the therapeutic treatment of iron homeostasis diseases or conditions associated with high levels of hepcidin in circulation, such as chronic disease anaemia.

An aspect of the present invention is therefore a sulphated glycosaminoglycan for use in the therapeutic treatment of a disease or condition with iron homeostasis disorders such as, preferably, an anaemia in a subject having high levels of serum hepcidin.

According to a preferred embodiment, the subject is a mammal, preferably a human being.

The person skilled in the art is perfectly capable of establishing whether the serum hepcidin levels measured in a patient who is affected by a disease or condition with iron homeostasis disorders, such as for example an anemia, are comprised within the ranges of values which are considered as normal or whether, in contrast, they represent a high level. The normal serum hepcidin levels are actually known to the skilled in the art. For example, if the subject is a human being, the expression "high levels of serum hepcidin" means a value which is higher than about 11 nM, as measured e.g. by mass spectroscopy techniques. It is to be understood that the technique for measuring serum hepcidin levels is not a limiting feature, rather it is provided only as an example.

Further embodiments of the invention are defined in the appended dependent claims.

The independent and dependent claims form an integral part of the description.

The invention is described in more detail in the experimental part which follows, with reference to the appended drawings, in which:

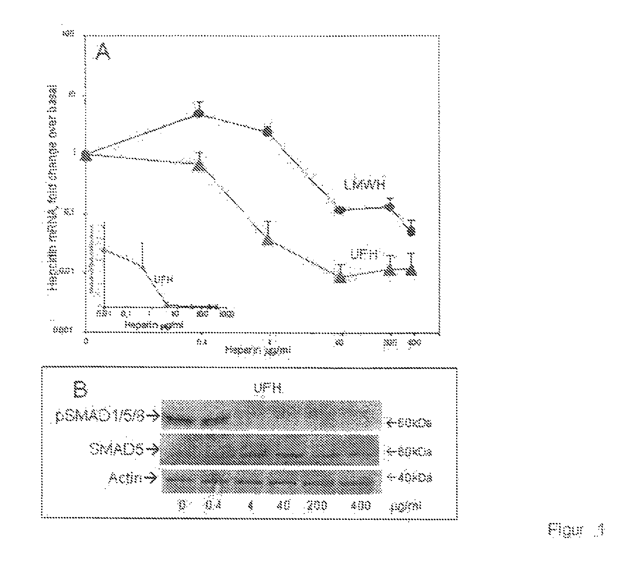

Figure 1A is a graph showing the inhibition of hepcidin mRNA by heparin. HepG2 cells were incubated for 16 hours with the concentrations of unfractioned heparin (UFH) and low molecular weight heparin (LMWH) shown in figure and the level of hepcidin mRNA was assessed in real time PCR (qRT-PCR)in relation to that of HPRT1. The data are expressed as a percentage in logarithmic scale in relation to the untreated cells. The insert shows the inhibition by UFH in a linear scale. Figure 1B shows a Western Blotting of phosphoSMAD1/5/8 of total SMAD 5 and of beta-actin as a calibrator of the cellular extracts after incubation with UFH.

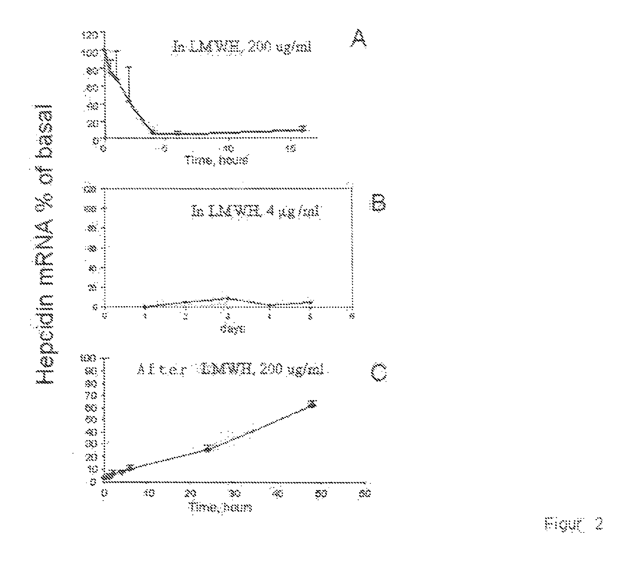

Figure 2 shows graphs illustrating the kinetics of the levels of hepcidin mRNA in HepG2 cells after the addition of LMWH. A: The cells were incubated with 200 µg/ml of LMW heparin for the time shown and the levels of hepcidin mRNA were measured. B: the cells were incubated with 4 µg/ml of LMW heparin for the time shown, changing the culture medium containing LMW heparin every two days, and the level of hepcidin mRNA was measured. C: the cells were incubated for 16 hours with 200 µg/ml of LMW heparin, rinsed and then grown for the time shown in a heparin-free medium and the levels of hepcidin mRNA were measured. Averages and SD of three independent experiments performed in triplicate.

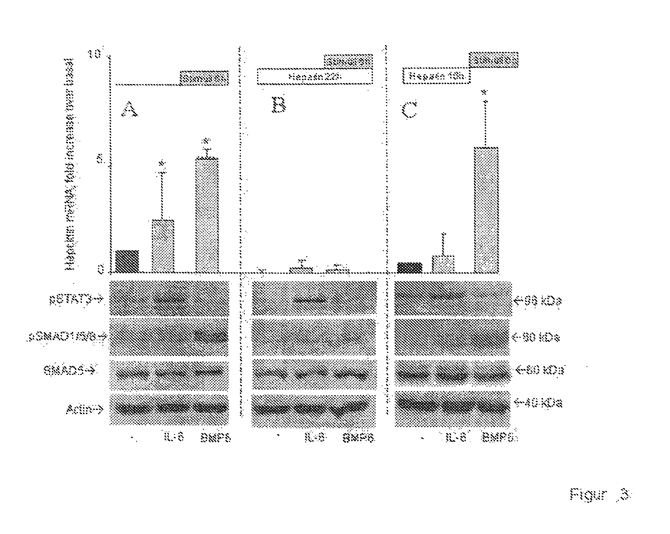

Figure 3 shows the effect of heparin on hepcidin induction by BMP6 and IL-6. Panel A: the HepG2 cells were grown for 6 hours with IL-6 (50 ng/ml), or BMP6 (10ng/ml) and then the mRNA hepcidin level was quantified by real time RT-PCR using HPRT1 mRNA as a reference. Panel B: as in A, except that the cells were first incubated for 16 hours with 200 µg/ml LMWH, then added with IL-6 or BMP6 and further incubated for 6 hours in the presence of LMWH. Panel c: as in B, except that the cells were first washed before enriching with IL-6 or BMP6 and incubated for 6 hours in the absence of heparin. The level of phosphoSTAT-3, phosphoSMAD1/5/8 and SMAD5 was measured by Western blotting and actin was used as a calibrator. Averages and SD of three independent experiments performed in triplicate. The asterisks indicate significant differences with respect to the unstimulated control.

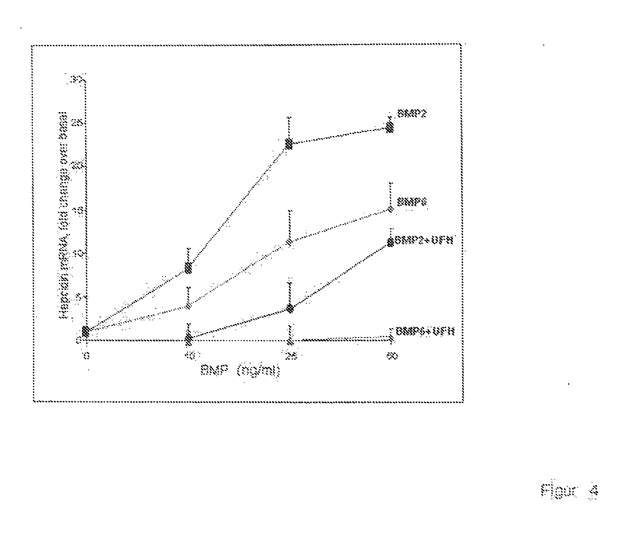

Figure 4 shows the effect of heparin on hepcidin induction by BMP2 and BMP6. HepG2 cells were grown for 16 hours at the BMP2 or BMP6 concentrations shown in the figure, in the presence or absence of 4 µg/ml UFH, and the hepcidin transcript level was evaluated by qRT-PCR.

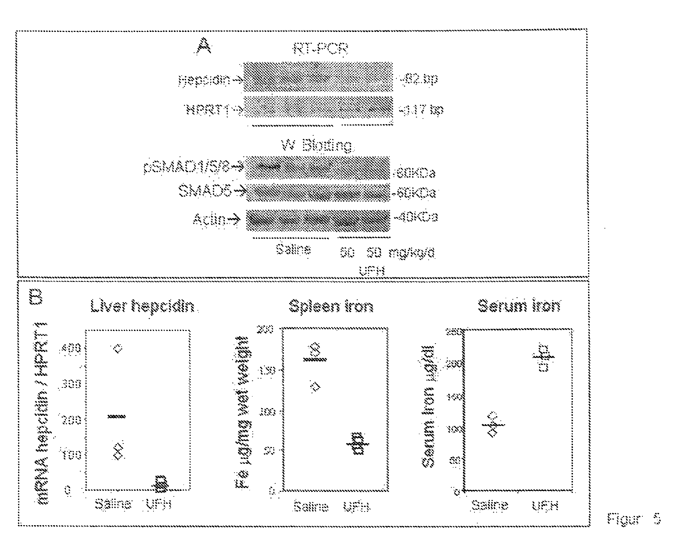

Figure 5 shows the in vivo effect of heparin injections. A: the mice were given subcutaneous injections of heparin or saline solution of 50mg/kg/day or saline for a week and the level of mRNA hepcidin in the liver was estimated by RT-PCR with respect to that of HPRT1. In the lower part of the figure, the phosphoSMAD1/5/8, SMAD5 and actin levels were analysed by Western blotting. B: mice were treated for 15 days with the pharmacological concentration of 2 mg/kg/d UFH or saline and the hepatic level of hepcidin mRNA was analysed by qRT-PCR with reference to the HPRT1 level. The animals were also analysed for Spleen iron concentration and Serum iron concentration. Results of three independent experiments.

The experimental part which follows is provided merely by way of a non-limiting example of the scope of the invention as defined in the appended claims.

### EXPERIMENTAL PART

### Introduction

Bone morphogenic proteins (BMPs) were originally identified for their ability to induce the ectopic formation of bone when implanted in muscular tissue and were isolated using heparin affinity columns. Over 15 BMPs have been identified in vertebrates and are classified into many sub-groups, among which BMP-2/4 and BMP-5/6/7. The proteins belonging to these two sub-groups have different structural properties. Most BMPs are expressed as inactive proteins which form homodimers or heterodimers and are secreted as active molecules after proteolytic processing. BMPs are widely expressed and their functions involve various development processes. In most conditions, BMPs act as morphogenes. Many recombinant BMPs have been found to be active in ectopic bone formation dosages and BMP2, BMP4 and BMP7 have been widely analysed for their ability to induce osteoblastic differentiation of myoblasts C2C12 or osteoblastic cells (ROS 17/2.8 or SaOS-2 cells). BMPs signalling is begun by the bond with specific type I and type II receptors which cause the phosphorylation of SMAD-1, 5 and 8. They form a complex with SMAD4 which moves into the nucleus and regulates the expression of the genes in co-operation with other transcription factors. BMPs activity is modulated in a complex manner by various factors. Some secretory molecules bind BMPs and prevent their interaction with the receptors on the cell surface, acting as antagonists; these include noggin, chordin, follistatin and members of the DAN family. In addition, heparan sulphate proteoglycans can bind BMPs, their antagonists and their receptors and modulate BMPs functioning in a complex manner. Soluble heparin has also been found to bind BMPs and contribute to regulating how they function. The alteration of endogenous heparan sulphates of ROS 17/12.8 cells with chlorate or heparanase treatment reduces the BMP7 bond and BMPs signalling. However the same treatment of C2C12 cells increases the morphogenic bioactivity of BMP2. Treatment with exogenous heparin in some cases had an effect on BMPs activity. Such treatment inhibited BMP7 and BMP6 signalling, while potentiating the osteogenic activity of BMP-2 and -4. Other studies have shown that heparin inhibits the osteogenic activity of BMP2 by binding to the BMPs receptors. In addition it has been shown that low concentrations of heparin facilitate the osteogenesis of Saos-2 cells, while high concentrations inhibit it. The effect of heparin is further complicated by its high bonding affinity with BMPs inhibitors such as chordin and noggin and its ability to displace these proteins from the cell membrane.

More recently it has been shown that BMPs are implicated in the systemic regulation of iron homeostasis, promoting the hepatic expression of hepcidin. This is a peptide expressed as a pre-pro-hormone of 84 amino acids which is processed in its active form of 25 residues. The receptor of hepcidin is ferroportin, the sole cellular iron exporter, which regulates iron absorption and recycling. Binding with hepcidin induces ferroportin degradation, thus inappropriately low levels of hepcidin are associated with iron overloads and various forms of genetic haemochromatosis, while high levels of hepcidin are found in inflammatory states and are considered an important cause of anaemia. The expression of hepcidin in hepatic cells is highly stimulated by all the BMPs tested so far, but BMP6 is likely its physiological regulator, since its expression is iron-dependent, and the BMP6 knock-out mice showed a high iron overload. Hepcidin expression is controlled by BMP/SMAD signalling, and hemojuvelin (HJV) is a BMPs co-receptor acting as a key regulator of hepcidin expression thereby facilitating SMAD signalling. Hepcidin is also stimulated by IL-6, by a signalling pathway which involves STAT-3, but which is dependent on the presence of SMAD4.

The role of heparin and heparan sulphate on BMP signalling in HepG2 hepatic cell lines expressing hepcidin has been studied by the present inventors. It has been found that heparin suppresses the basal expression of hepcidin at surprisingly high levels and that it also suppresses the introducton of hepcidin by BMP6 and BMP2. Moreover, surprisingly, heparin also inhibits the induction of hepcidin by the inflammatory cytokine IL-6. Moreover, in vivo experiments have shown that subcutaneous injections of heparin at pharmacological levels strongly inhibited the expression of hepcidin in the liver of mice and, surprisingly, also reduced systemic bioavailability of iron.

### Materials and methods

*Cell cultures*. The HepG2 cells were grown in Minimal Essential Medium (MEM) (Life Technologies, Invitrogen, Bethesda, MD) with 10% foetal calf serum (FCS, Clontech, Palo Alto, CA), 40 µg/ml gentamicin and 1 mM L-glutamine). The cells were kept at 37°C with 5% CO₂.In phosphorylation or SMAD activation studies, 10⁵ cells/well were seeded in 12-well plates, grown for 24 hours and then for another 16 hours in 0.5%FCS. They were then incubated with recombinant BMP2 (R&D Systems), BMP6 (R&D Systems), IL-6 (R&D Systems), heparin (Eparina Vister or Calciparina, Italfarmaco) or low molecular weight heparin (Clexane, Sanofi Aventis).

*Immunoblotting.* The cell extracts were lysed in lysis buffer (200mM Tris-HCI pH8, 100mM NaCl, 1mM EDTA, 0.5%NP-40 and 10% glycerol) containing a mixture of protease inhibitors (Sigma). For dosages of the phosphorylated SMADs, 1 mM sodium orthovanadate (Sigma) and 1mM sodium fluoride (Sigma) were added to the lysis buffer as phosphatase inhibitors.

The cell lysates were analysed on 10% SDS-PAGE and, after transfer, the nitrocellulose filters were incubated for 16 hours with specific antibodies, rinsed and incubated further for 1 hour with peroxidise-labelled secondary antibodies (Dako anti-mouse IgG, Glostrup, Denmark or Pierce anti-rabbit IgG). The following were used as primary antibodies: rabbit anti-phosphoSMAD1/ 5/8 antibody (1:1000; Cell Signalling Technology), rabbit anti-SMAD5 antibody (1:1000 Cell Signaling Technology), mouse anti phosphoSTAT-3 antibody (1:1000, Cell Signalling Technology) and rabbit anti-actin antibody (1:1000; Sigma). Bound activity was determined using an ECL kit(Amersham, Uppsala, Sweden) and revealed using a KODAK Image Station 440CF (Kodak, Rochester, NY).

*RNA extraction and RT-PCR.* The RNA was purified from the cells or from mouse livers using the guanidium thiocyanate-phenol-chloroform method (Trizol) according to the manufacturer's instructions (Ambion, Austin, Tx). Total RNA treated with DNase (1 µg) was used to synthesise the first cDNA strand with ImProm-II Reverse Transcription System (Promega), using oligo dT. For RT-PCR analysis, the assays-on-Demand products (20x) and TaqMan Master Mix (2x) of Applied Biosystems (Foster City, CA) were used according to the manufacturer's instructions and the reactions were run on an ABI PRISM 7700 Sequence Detection System (Applied Biosystems) in a final volume of 20 µl for 40 cycles. Hepcidin expression levels were normalised to those of HPRT1 in each sample.

*Mice.* The mice were given daily subcutaneous injections of saline solution, heparin or low molecular weight heparin. After seven days they were sacrificed and the livers homogenised and analysed for the hepcidin transcript or for phosphoSMAD1/5/8 and SMAD5, using actin as a control. For Western Blotting analysis, the antibodies mentioned above have been used. The following primers were used for RT-PCR analysis:

mHPRT1-for 5'- GCTTGCTGGTGAAAAGGACCTCTCGAAG -3'

mHPRT1-rev 5'- CCCTGAAGTACTCATTATAGTCAAGGGCAT -3'

mHEPC1-for: 5'- CTGTCTCCTGCTTCTCCTCCTT -3'

mHEPC1-rev: 5'- CTGCAGCTCTGTAGTCTGTCTCATC -3'

For Real Time RT-PCR analysis mouse-specific Assays-on-demand products (20x) and TaqMan Master Mix (2x) (Applied Biosystems) were used.

*Statistical analysis.* A comparison of the untreated control (mock) cells and the treated cells was performed using the Student t test for unpaired data. Differences with P values below 0.05 were defined significant.

Results

Heparin inhibits hepcidin expression in HepG2 cells.

BMPs and in particular BMP6, are the most important hepatic hepcidin regulators, thus it was of interest to evaluate how this regulatory pathway was influenced by exogenous heparin. The inventors used human hepatoma cells HepG2, a model widely used to study hepcidin expression. In initial experiments the cells were treated for 16 hours with different concentrations of heparin (from 0.4 to 400µg/ml) and the level of hepcidin mRNA was analysed using Real Time RT-PCR. An inhibition curve was obtained with a reduction in the hepcidin transcript to a level of about 1/100 of the baseline at concentrations of over 4 µg/ml (figure 1). Similar inhibition curves were obtained after 48 hours of incubation (data not shown). The inventors then analysed a preparation of low molecular weight (LMW) heparin in the same concentration range. In this case too an inhibition of about 10-folds at concentrations over 40 µg/ml LMWH (figure 1) was obtained. The comparison betwen the two curves shows that LMW heparin is not as strong as heparin in inhibiting hepcidin expression. The inventors then performed time trend experiments with LMW heparin at the highly inhibitory concentration of 200µg/ml. Hepcidin mRNA decreased linearly in the first 4 hours and then levelled out at values below 10% of the baseline (figure 2A). The effect was quite rapid and already evident after 30 minutes of incubation. To assess the long term effects, the cells were grown for up to seven days in 4 µg/ml of LMW heparin, changing the culture medium every two days. The data confirms that at this low concentration of heparin, the hepcidin mRNA was strongly inhibited to values below 10% of the baseline and showed that repression lasted for the entire incubation period (figure 2B). It had no effect on cell proliferation and vitality (data not shown). To assess the duration of the inhibitory effect, the cells were grown for 16 hours in 200 µg/ml LMW heparin, rinsed and grown further in a heparin-free medium for another two days. The hepcidin mRNA level increased slowly from the initial 3% to about 60% of the baseline in 48 hours (figure 2C). The inventors have concluded that both heparin and LMW heparin have a strong inhibitory activity on hepcidin expression, which acts in a few hours and persists for days.

Heparin inhibits the induction of hepcidin by BMP6 and IL-6.

The inventors have confirmed that treatment of 6 hours with 10ng/ml of BMP6 causes a three to four-fold induction of hepcidin mRNA and with 50ng/ml of IL-6 a 2-3 fold induction (figure 3A). In further experiments, the cells were incubated for 16 hours in 200 µg/ml LMW heparin to reduce the hepcidin mRNA level, and then were treated with BMP6 or IL-6 in the presence of LMW heparin. The treatment with BMP6 and with IL-6 caused a marginal increase of about 15-20% (figure 3B). It is known that BMPs act on hepcidin expression by means of SMAD signalling, which includes the phosphorylation of the SMAD1/5/8. Western blotting analysis of the cell homogenates showed that treatment with LMW heparin reduced the baseline level of phosphorylated SMAD, that treatment with IL-6 had no effect, while treatment with BMP2 and BMP6 caused strong induction (figure 3). More interestingly, in the cells treated for 16 hours with LMW heparin the level of phosphoSMAD1/5/8 remained repressed after 4 hours of treatment with IL-6 and BMP6, in parallel with hepcidin expression. The phosphorylation of STAT-3 was not influenced by LMW heparin, and was induced only by IL-6, to the same extent before and after incubation with heparin LMW (figure 3). The level of phospho Erk1/2 was also analysed, and it was found that it is not influenced by LMW heparin (data not shown). In a further experiment, the cells were treated for 16 hours with 200 µg/ml LMWH, washed and then added with IL-6 or BMP6. Surprisingly, BMP6 caused hepcidin induction similar to that in the untreated cells, while IL-6 caused only a marginal induction.

To study whether heparin has a comparable effect on the two BMPs which are more studied, the cells were incubated for 16 hours with varying concentrations of BMP2 and BMP6 in the presence or absence of 4 µg/ml heparin and then the hepcidin mRNA level was analysed. The data in figure 4 show that, surprisingly, heparine has a lower inhibitory effect on BMP2 activity than on BMP6 activity.

Heparin inhibits in vivo hepatic hepcidin expression.

The inventors then analysed the in vivo effect of heparin. The mice were treated with daily injections of various doses of heparin or LMW heparin for one week and the liver and serum were analysed. RT-PCR analysis showed a marked reduction of hepcidin mRNA in the liver after treatment with 50mg/kg once a day (figure 5A). This was accompanied by a strong reduction in phosphoSMAD1/5/8. In other experiments, the mice were treated for two weeks with 2mg/kg a day of UFH. Surprisingly, it was observed that also this physiological treatment with heparin is sufficient to stongly reduce the level of hepcidin in the liver measured using RT-PCR, to reduce iron in the spleen and to increase iron in serum (figure 5B).

Discussion

The inventors have demonstrated that heparin and LMW heparin inhibit hepcidin expression in hepatoma HepG2 cells in a dose dependent manner. The effect is evident after 30 minutes of incubation and complete after 4 hours, after which it persists for up to one week. Moreover, after removal of the heparin, the inhibition slowly decreases and the level reaches 60% of the baseline in about two days. It has been demonstrated that LMW heparin is less potent than unfractionated heparin.

It has been observed that heparin reduces the baseline level of phosphoSMAD1/5/8 and that it inhibits the induction of phosphorylation of the SMAD caused by BMP6. This indicates that it influences hepcidin expression by interfering with the signalling pathway BMP/SMAD. Many other molecules involved in this pathway bind heparin, including the BMP receptor, BMP inhibitors such as noggin or chordin and the BMP molecules themselves. The data present is not sufficient to establish which of them is the one mainly responsible for inhibition, but some indications may be obtained from the unexpected discovery that after having removed heparin by washing, addition of BMP6 completely abolishes hepcidin inhibition, suggesting that heparin sequestrates BMP6. Another unexpected result is the evidence that heparin inhibits BMP6 induction but not BMP2 induction. These proteins belong to two separate groups of BMPs. BMP2 and BMP4 are characterised by an amino-terminal heparin-binding domain, and the osteogenic and SMAD activities of BMP2 and 4, are stimulated by low concentrations of heparin apparently in an indirect manner acting on the stabilisation of the BMPs. BMP6 and BMP7 belong to another sub-group of BMPs, which lacks the heparin-binding amino-terminal domain, but despite this BMP7 and BMP6 have a high binding affinity with heparins. In addition, the osteogenic activity of BMP7 and BMP6 was also inhibited by low concentrations of exogenous heparin. All these data indicate that BMP2 and BMP6 can bind heparin in different ways, and that these proteins can recognise different receptors. In fact, the inactivation of the receptor II of the BMPs (BMPR2) reduces the signal from BMP2 and BMP4, but increases that of BMP6 and BMP7.

IL-6 produces reduced hepcidin induction and does not abolish the inhibition caused by heparin. It has been shown that IL-6 acts through activation of STAT-3 which binds an element of the hepcidin promoter near the putative one of SMAD4 and which needs SMAD4 to induce hepcidin. Heparin does not modify the phosphorylation of STAT-3 but its inhibition of the SMAD pathway is probably sufficient to reduce the functionality of STAT-3. All together these data indicate that heparin inhibits the signalling pathway for hepcidin regulation which includes BMP6 and IL-6, the two physiological hepcidin regulators.

The inventors have also shown that heparin and LMW heparin have a strong inhibitory activity on the expression of hepcidin in vivo. The mice were treated with therapeutic doses of heparin and the reduction of hepcidin in the liver was dramatic, to 2-5% of that of the control animals. In addition, these treatments also resulted in increased serum iron and decreased iron concentration in the spleen. This is in complete agreement with the role of hepcidin whose reduction, caused by heparin treatment, increases the release of iron from spleen microphages and increases bioavailability of iron in serum. This opens the way for clinical use of heparin to reduce hepcidin in subjects suffering from anaemia.

To conclude, the experimental results obtained by the inventors show that sulphated glycosaminoglycans are deeply involved in the signalling pathway of the BMPs in hepatic cells which regulates hepcidin expression. The role of the endogenous heparan sulphates still needs to be analysed, as well as the relationship between heparin and the various molecules which bind heparin involved in the signalling pathway. However, the most important discovery is that exogenous heparin is a strong inhibitor of the expression of hepcidin in vitro and in vivo. An abnormal increase of hepcidin often occurs in inflammatory states and in chronic inflammations and is thought to be the cause of the reduced absorption and recycling of iron by the macrophages to the erythroid cells causing anaemia. In IRIDA anaemia (Iron Refractory Iron Deficiency Anaemia), mutations of the matriptase 2 gene alter HJV functioning with a consequent increase of hepcidin. This causes iron deficiency and anaemia. The results obtained by the inventors indicate that heparin and the derivatives thereof, such as modified heparins with no anti-coagulant activity, may be useful in the therapeutic treatment of both types of disease.

To such purpose, heparin or the derivatives thereof may be formulated in any pharmaceutical form suitable for subcutaneous or intravenous administration, said pharmaceutical form containing a pharmaceutically effective amount of active ingredient and using pharmaceutical excipients known per se. T h e pharmacologically effective amount of active ingredient depends on the characteristics of the disease to be treated and of the patient, but is usually comprised within the range of 1-200mg/kg/day.

## Claims

1. A sulphated glycosaminoglycan for use in the therapeutic treatment of a disease or condition with iron homeostasis disorders in a subject having high levels of serum hepcidin.

2. The sulphated glycosaminoglycan for use according to claim 1, wherein the disease or condition is an anaemia.

3. The sulphated glycosaminoglycan for use according to claim 2, wherein the anaemia is chronic disease anaemia.

4. The sulphated glycosaminoglycan for use according to any of claims 1 to 3, which is selected from the group consisting of heparin, low molecular weight heparins and natural or biotechnological derivatives thereof with reduced anticoagulant activity or with no anticoagulant activity.

5. The sulphated glycosaminoglycan for use according to any of claims 1 to 4, which is formulated as a medicament suitable for subcutaneous or intravenous administration.

6. The sulphated glycosaminoglycan for use according to any of claims 1 to 4, wherein the subject is a mammal, preferably a human being.

## Patentansprüche

1. Sulfatiertes Glycosaminoglycan zur Verwendung in der therapeutischen Behandlung einer Erkrankung oder eines Zustandes mit Eisen-Homöostase-Störungen in einem Subjekt mit hohem Hepcidinspiegel im Serum.

2. Sulfatiertes Glycosaminoglycan zur Verwendung nach Anspruch 1, wobei die Krankheit oder der Zustand eine Anämie ist.

3. Sulfatiertes Glycosaminoglycan zur Verwendung nach Anspruch 2, wobei die Anämie eine chronische Anämieerkrankung ist.

4. Sulfatiertes Glycosaminoglycan zur Verwendung nach einem der Ansprüche 1 bis 3, welches aus der Gruppe, bestehend aus Heparin, Heparinen mit niedrigem Molekulargewicht und deren natürlichen oder biotechnologischen Derivaten mit reduzierter gerinnungshemmender Aktivität oder ohne gerinnungshemmende Aktivität, ausgewählt ist.

5. Sulfatiertes Glycosaminoglycan zur Verwendung nach einem der Ansprüche 1 bis 4, welches als ein Medikament formuliert ist, das zur subkutanen oder intravenösen Verabreichung geeignet ist.

6. Sulfatiertes Glycosaminoglycan zur Verwendung nach einem der Ansprüche 1 bis 4, wobei das Subjekt ein Säugetier, vorzugsweise ein Mensch, ist.

## Revendications

1. Glycosaminoglycane sulfaté pour utilisation dans la prise en charge thérapeutique d'une maladie ou d'une affection associée à des troubles de l'homéostasie du fer chez un sujet ayant des taux d'hepcidine sérique élevés.

2. Glycosaminoglycane sulfaté pour utilisation selon la revendication 1, dans lequel la maladie ou l'affection est une anémie.

3. Glycosaminoglycane sulfaté pour utilisation selon la revendication 2, dans lequel l'anémie est une anémie pathologique chronique.

4. Glycosaminoglycane sulfaté pour utilisation selon l'une quelconque des revendications 1 à 3, qui est choisi dans le groupe constitué par l'héparine, les héparines de bas poids moléculaire et leurs dérivés naturels ou biotechnologiques ayant une activité anticoagulante réduite ou sans activité anticoagulante.

5. Glycosaminoglycane sulfaté pour utilisation selon l'une quelconque des revendications 1 à 4, qui est formulé sous forme de médicament acceptable pour une administration par voie sous-cutanée ou intra-veineuse.

6. Glycosaminoglycane sulfaté pour utilisation selon l'une quelconque des revendications 1 à 4, dans lequel le sujet est un mammifère, de préférence, l'homme.
